# EUROPEAN PATENT APPLICATION

(11) **EP 1 627 660 A1**
(43) Date of publication of application: **22.02.2006**
(21) Application number: 04090408.8
(22) Date of filing: 25.10.2004
(51) Int. Cl.: A61N 1/362, A61N 1/39

(54) **Two lead three-chamber pacing system for CHF patients**

(30) Priority: 20.08.2004 US 603441 P
(71) Applicant: Biotronik GmbH & Co. KG, 12359 Berlin (DE)
(72) Inventor: Thong, Tran, Portland Oregon 97229 (US); Schaldach, Max, deceased (DE)
(74) Representative: Eisenführ, Speiser & Partner

(57) **Abstract**

A pacing system for a human heart has a pacing device,, communicated to first and second electrical lead arrangements. The first electrical lead arrangement is suitable for placement through the superior vena cava and right atrium into the right ventricle and the second electrical lead arrangement suitable for positioning into the left ventricle and communicated to the pacing device. The second electrical lead arrangement is sized and manufactured to be suitable for insertion into the superior vena cava of the heart, through the coronary sinus communicated thereto, and navigable through a vein turning off of the coronary sinus into the left ventricle.

## Description

The present invention relates to a device for pacing a heart of a human patient, particularly one having congestive heart failure (CHF). More particularly, the device comprises two leads for pacing three chambers of the patient's heart.

In the medical condition known as congestive heart failure, the patient has an acute or chronic disability to properly oxygenate the body, due to a diminished blood flow from the heart. One result is a pooling of excess blood in the organs, since the heart does not recirculate the full amount of blood available through the great veins. In many CHF cases, the patient needs left ventricular support, which is conventionally provided by introducing a thin catheter into the coronary sinus, which is the primary receptor of blood supplied to nourish the walls and muscles of the heart. This catheter is inserted through the great cardiac vein, which drains the anterior aspect of the heart. This catheter is intended to stimulate the left ventricle.

To be effective, left ventricle stimulation must be synchronized with right ventricle stimulation. Similarly, the respective ventricular stimulations must be synchronized with the atrial contraction, especially the left atrial contraction, to provide the effect known as "atrial kick", It should be readily understood that, in this context, the term "synchronized" is not synonymous with the term "simultaneous," as there will be situations where a delay is not only tolerable, but is actually preferred.

Many patients who require cardiac pacing of this type also may need defibrillation. For that reason, it is known to incorporate both a pacemaker and an implantable cardioverter defibrillator ("ICD") into a single device. Such a combined device is often referred to as an implantable cardioverter defibrillator with biventricular pacing capability. In such a device, the prior art would provide the device with three electrical leads or catheters, which would be introduced in to the heart through the veins. The first lead would be a right ventricular lead for pacing and sensing. In the situations where the device is an ICD system, this first lead would also provide at least a first shock coil for location in the right ventricle and possibly a second shock coil for location in the vena cava superior. The second lead would pace and sense in the right atrium. The third lead would pace and sense in the left ventricle.

This third lead is typically inserted into the heart through the coronary sinus through the great cardiac vein.

The use of a single-lead electrode arrangement in the right side of the heart was disclosed in German published patent application 196 26 352, published 6 June 1996, by Thong and Schaldach, This electrode arrangement provides two right atrial ring electrodes, one right ventricular tip electrode, one right ventricular ring electrode and one right ventricular shock coil Such an electrode arrangement may be introduced through the vena cava superior into the right atrium and right ventricle. Access to the left side of the heart is different than access to the right side, so the known electrode arrangements for the right side are simply not applicable to the left side.

It is always a delicate and risky procedure to insert leads into the heart. Also, when previous pacemaker implants are replaced, it is customary to not have to remove abandoned leads from the prior implants. It is an unmet need in the prior art to reduce the number of leads which must be inserted into the heart below the three leads described above.

To meet hat need, according to the invention a pacing system is suggested that comprises a pacing device, a first electrical lead arrangement suitable for placement through the superior vena cava and right atrium into the right ventricle and communicated to the pacing device and a second electrical lead arrangement suitable for positioning into the left ventricle and communicated to the pacing device.

It is, therefore, an advantage of the present invention to combine the functionality of the second and third leads described above in a single lead, while providing an arrangement that is able to be navigated into position from the vena cava superior into the coronary sinus and from there into the left ventricle through a vein turning off from the coronary sinus. Such an advantage is achieved by a pacing system for a human heart having a left and a right atrium and a left and a right ventricle.

In other aspects of the invention, the second electrical lead arrangement is suitable for insertion into the superior vena cava of the heart, through the coronary sinus communicated thereto, and navigable through a vein turning off of the coronary sinus into the left ventricle.

In further embodiments of the invention, the second electrical lead arrangement comprises at least two ring electrodes along an intermediate portion thereof and positionable in the coronary sinus for electrical communication with the left atrium, and a tip electrode at a distal end thereof, suitable for insertion into the left ventricle.

In other embodiments,, the second electrical lead arrangement further comprises
a shock coil or a ring electrode near the distal end thereof, suitable for insertion into the left ventricle.

In yet further embodiments, the first electrical lead arrangement further comprises first and second shock coils along the length thereof, the first shock coil for positioning in the right atrium and the second for positioning in the right ventricle.

The present invention is better understood when reference is made to the accompanying drawings, in which identical parts are indicated by identical reference numerals, and wherein:
- FIGURE 1: shows a first embodiment of the present invention,, deployed in a human heart;
- FIGURE 2: shows a second embodiment of the present invention, deployed in a human heart; and

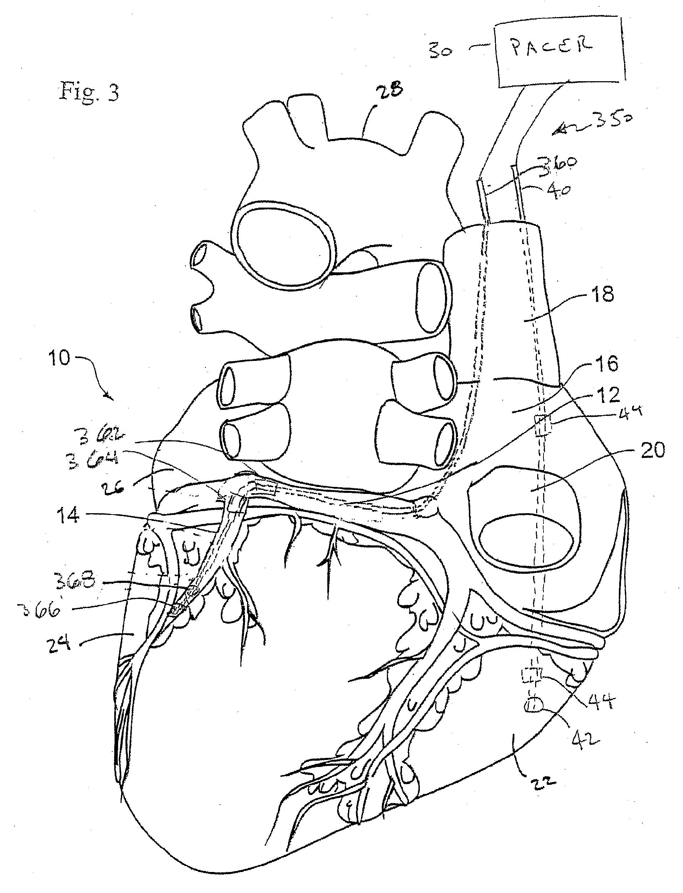

- FIGURE 3: shows a third embodiment of the present invention, deployed in a human heart.

The present invention is best understood when shown in the context of a human heart in which it would be intended to be implanted. For that purpose, the appended figures show the embodiments of the invention with respect to a heart 10, viewed in a posterior external manner. In the figures, the following structures are shown: the coronary sinus 12; the lateral vein 14; the right atrium 16; the superior vena cava 18; the inferior vena cava 20; the right ventricle 22; the left ventricle 24; the left atrium 26 and the aorta 28. Because the anterior view of the heart is shown, the left side of the heart lies to the left in the figures and the right side of the heart lies to the right in the figures. In each of the figures, a pacing device 30 as known in the prior art is shown schematically as connected to the electrode leads of the present invention.

It is commonly known to access the heart 10 with electrode feeds that enter through the superior vena cava 18, lying atop the right atrium 16. For this reason, a portion of each embodiment taught herein will include a right side lead 40, equipped with at least one electrode 42 toward the distal end thereof for right ventricular sensing. Because this right side lead 40 is in the chambers of the heart, it can be larger and less flexible than a left side lead.

Access to the left side of the heart 10 is more difficult. To gain access to the left side via the superior vena cava 18, an electrode lead must possess sufficient flexibility and must be narrow enough to pass through an opening 32 in the right atrium into the coronary sinus 12, From thence, the electrode must pass along the coronary sinus 12 to a lateral vein 14, which leads downwardly and into the left ventricle 24. Note that this electrode does not enter the left atrium 26, so electrical communication with the left atrium must be established by electrodes in the coronary sinus as it passes on the surface of the left atrium.

The present invention exists in at least three different embodiments.

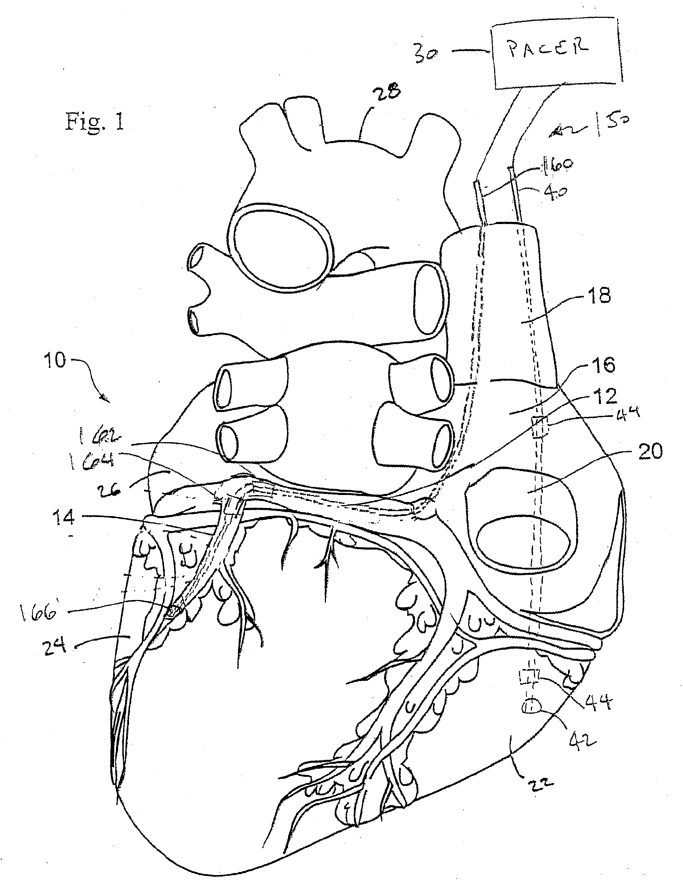

In a first embodiment 150 and shown in Figure 1, the first lead is the standard right side lead 40 with at least one right ventricle electrode 42. If defibrillation capacity is desired, then at least one shock coil 44 can be provided in the right ventricle, the right atrium or both. The second lead 160, of a diameter, flexibility and material suitable to pass through the coronary sinus, has at least a pair of ring electrodes 162, 164 for use in association with the left atrium 26 and a tip electrode 166 for use in association with the left ventricle 24. Accordingly, three chambers of the heart are reached by two associated electrode leads.

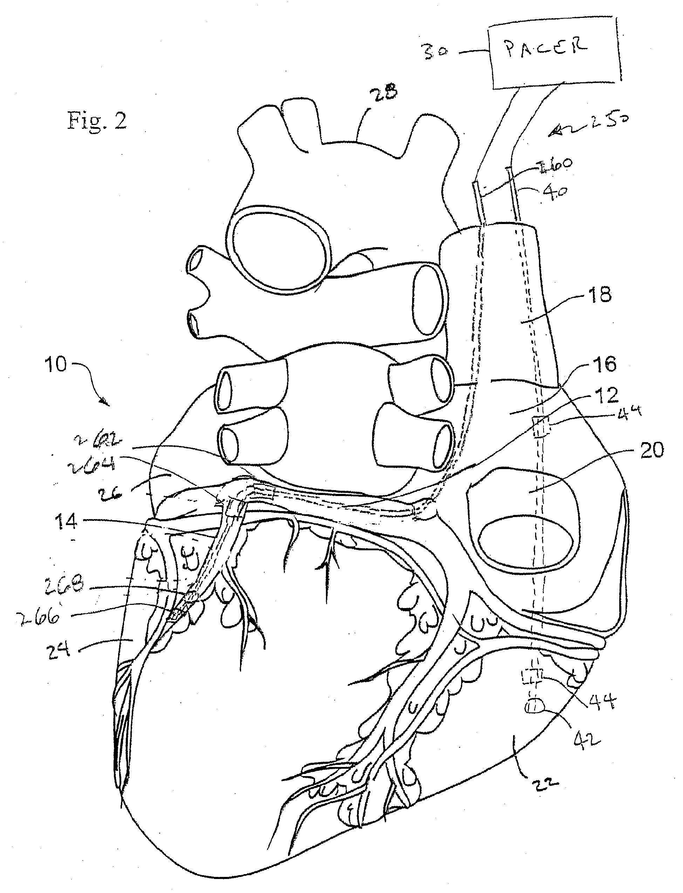

In a second embodiment: 250 and shown in Figure 2, the first lead is the standard right side lead 40 with at least one right ventricle electrode 42. If defibrillation capacity is desired, then at least one shock coil 44 can be provided in the right ventricle, the right atrium or both. The second lead 260, of a diameter, flexibility and material suitable to pass through the coronary sinus, has at least a pair of ring electrodes 262, 264 for use in association with the left atrium 26, a tip electrode 266 for sensing in the left ventricle 24 and a shock coil 268 for pacing in the left ventricle. Accordingly, three chambers of the heart are reached by two associated electrode leads.

In a third embodiment 350 and shown in Figure 2, the first lead is the standard right side lead 40 with at least one right ventricle electrode 42. If defibrillation capacity is desired, then at least one shock coil 44 can be provided in the right ventricle, the right atrium or both. The second lead 360, of a diameter, flexibility and material suitable to pass through the coronary sinus, has at least a pair of ring electrodes 362, 364 for use in association with the left atrium 26, a tip electrode 366 for sensing in the left ventricle 24 and a ring electrode 368 for sensing in the left ventricle. Accordingly, three chambers of the heart are reached by two associated electrode leads.

Stated succinctly, the present invention relies upon several aspects. The invention provides left atrial sensing by using two ring electrodes in the coronary sinus. These two ring electrodes allow pacing in the left atrium. Left ventricle sensing is possible from the left ventricle tip electrode to the right ventricle ring electrode. Another option for left ventricle sensing is to use the two ring electrodes in the distal coronary sinus and great cardiac vein. These left ventricle sensing options may not be of particular importance, because many cardiologists do not consider left ventricle sensing itself to be of particular importance.

It is also possible to achieve left ventricle pacing from the left ventricle tip electrode to the right ventricle ring electrode, A second option for left ventricle pacing would be through the two ring electrodes in the distal coronary sinus and great cardiac vein.

## Claims

1. A pacing system for a human heart having a left and a right atrium and a left and a right ventricle, the pacing system comprising:
a pacing device;
a first electrical lead arrangement suitable for placement through the superior vena cava and right atrium into the right ventricle and communicated to the pacing device; arid
a second electrical lead arrangement suitable for positioning adjacent to the left ventricle and communicated to the pacing device, the second electrical lead arrangement including at least two ring electrodes along an intermediate portion thereof and being positionable in the coronary sinus for electrical communication with the left atrium, and including a tip electrode at a distal end thereof suitable for insertion into a vein near the left ventricle,

2. The pacing system of claim 1, wherein the second electrical lead arrangement Is suitable for insertion into the superior vena cava of the heart, through the coronary sinus communicated thereto, and navigable through a vein turning off of the coronary sinus into the left ventricle.

3. The pacing system of claim 1 or 2, wherein the second electrical lead arrangement further comprises:
a shock coil near the distal end thereof, suitable for insertion into a vein near the left ventricle.

4. The pacing system of one of claims 1 to 3, wherein the second electrical lead arrangement further comprises:
a ring electrode near the distal end thereof, suitable for insertion into a vein near the left ventricle.

5. The pacing system of one of claims 1 to 4, wherein the first electrical lead arrangement further comprises:
a first shock coil along the length thereof for positioning in the right atrium or the right ventricle.

6. The pacing system of claim 5, wherein the first electrical lead arrangement further comprises:
a second shock coil along the length thereof, such that one shock coil is positioned in the right atrium and the other shock coil is positioned in the right ventricle.

7. The pacing system of claim 3, wherein the first electrical lead arrangement further comprises:
a first shock coil along the length thereof for positioning in the right atrium or the right ventricle,

8. The pacing system of claim 7, wherein the first electrical lead arrangement further comprises:
a second shock coil along the length thereof, such that one shock coil is positioned in the right atrium and the other shock coil is positioned in the right ventricle.

9. The pacing system of claim 4, wherein the first electrical lead arrangement further comprises:
a first shock coil along the length thereof for positioning in the right atrium or the right ventricle.

10. The pacing system of claim 9, wherein the first electrical lead arrangement further comprises:
a second shock coil along the length thereof, such that one shock coil is positioned in the right atrium and the other shock coil is positioned in the right ventricle.
